# EUROPEAN PATENT APPLICATION

(11) **EP 2 623 152 A1**
(43) Date of publication of application: **07.08.2013**
(21) Application number: 13152867.1
(22) Date of filing: 28.01.2013
(51) Int. Cl.: A61M 37/00, B25G 1/10

(54) **Gripping device and method of use thereof**

(30) Priority: 01.02.2012 GB 201201687
(71) Applicant: Pierce, Jai Dickenson, Ryhill, Wakefield WF4 2QY (GB)
(72) Inventor: Pierce, Jai Dickenson, Ryhill, Wakefield WF4 2QY (GB)
(74) Representative: Tomkinson, Alexandra

(57) **Abstract**

A gripping device (2) is provided for gripping in a user's hand in use. The gripping device includes a body portion (3) having a first end (26) and a second end (28), and at least first, second and third digit location means (14, 16, 18) defined in the body portion between said first and second ends for the location of at least part of a user's hand digit in use. The first digit location means is provided along the length of the body portion between the first and second ends. The at least second and third digit location means are provided either side of the first digit location means.

## Description

This invention relates to a gripping device and to a method of use thereof.

Although the following description refers almost exclusively to a gripping device for use with a tattooing device, it will be appreciated by persons skilled in the art that the present invention could be used on any hand held device that would benefit from an ergonomic gripping or handle portion.

Tattoos have been used as an aesthetic form of a user's personal expression for hundreds of years throughout the world. A tattoo is formed by injecting indelible coloured ink into the dermis layer of a user's skin to cause a permanent change in the appearance of the skin pigment. Tattoos are typically provided according to a user's chosen image, word, number, symbol, pattern, design and/or the like. In the western world, a tattoo is typically applied using an electric tattooing device. The device inserts ink into the skin via a needle attached to an electrically powered oscillating unit. The unit rapidly and repeatedly drives the needle in and out of the user's skin 80-150 times/second. A tattooing tube is typically detachably attached to the oscillating unit and the tattooing tube includes an ink reservoir via which ink is applied to the user's skin for injection through the user's dermis via the oscillating needle. A gripping portion is provided on the tattooing tube to allow the device to be held by the tattooist in use.

It will be appreciated that the application of a tattoo onto a user via a tattooist is a highly skilled job requiring time, precision and care to ensure the tattoo being applied to the user is in the required form chosen by the user. It is therefore important for the tattooing device to sit comfortably in the tattooist's hand and to allow them to have sufficient control to easily manipulate the tattooing device to provide the required tattoo, particularly if the tattoo is an intricate design. In addition, a tattooist may work for between 8-10 hours a day using a tattooing device and therefore it is important for the gripping position of the tattooist's hand on the gripping portion to be comfortable. Many conventional tattooing devices are heavy, and do not allow a user to adopt a comfortable gripping position with the same, thereby resulting in the tattooist incurring repetitive strain related injuries to their digits, hand, wrist and forearm, particularly if the device is used for prolonged periods of time.

The gripping portion of many conventional tattooing devices typically comprises a cylindrical portion with ribs provided thereon to prevent the user's hand from slipping in use. Such gripping portions are not ergonomically designed and are typically uncomfortable to use for prolonged periods of time.

US2010/0206138 discloses a biodegradable and disposable tattoo tube. A shaped tip is formed at one end with an ink reservoir adjacent the tip. An ergonomic grip is disposed between opposing ends of the tube. The ergonomic grip has a single circumferential curved recess adjacent one end of the grip for the location of one or more of the user's fingers. The single recess does not maintain the position of the user's fingers in any pre-determined position relative to the grip or single recess and therefore a user's fingers may still slip during operation of the device.

US D609,339 shows a hand grip of a tattoo device that includes a number of finger recesses defined thereon. These finger recesses typically include a thumb recess provided substantially centrally of the device and perpendicular to the longitudinal axis of the tattooing device/tube, a finger recess located centrally of the device and perpendicular to the thumb recess, and two side recesses. Whilst this design provides some ergonomic features to try and address the abovementioned problem, the positions of the thumb and finger recesses do not conform to a typical user's grip. In particular, the position of the recesses widens the hand grip than is natural for most people, thereby making the handgrip more unstable and placing more strain on the user's hand. The design is for use by both left and right handed people but is not specifically designed for either handedness and therefore still lacks a desired level of comfort when gripping the same. In addition, the gripping portion is defined on a sleeve that fits over an existing tattooing tube. The hygiene requirements for tattooing are high due to the potential risk of spread of infection and therefore it is essential that the equipment can be easily dismantled for cleaning, sterilising and/or autoclaving purposes. The sleeve is difficult to fit and/or remove and this design therefore makes cleaning of the tattooing device difficult.

USD610614 shows an ergonomic writing instrument with one or more recesses for allowing a user's fingers and/or thumb to be located therein. However, the position of the one or more recesses is not clear and there only appears to be a clearly defined recess for location of a user's thumb therein. This device is therefore unlikely to offer the comfort required by a user for use of the device over a relatively long period of time.

Similar problems to those described above are encountered with other hand held devices. Such devices can include, for example, dental hand held devices, hand held laser devices, hand held medical devices, pens, engraving tools, paint brushes and/of the like.

It is therefore an aim of the present invention to provide a gripping device that has an improved ergonomic design and which overcomes the abovementioned problems.

It is a further aim of the present invention to provide a method of using a gripping device having an improved ergonomic design.

According to a first aspect of the present invention there is provided a gripping device for gripping in a user's hand in use, said gripping device including a body portion having a first end and a second end and at least first, second and third digit location means defined in said body portion between said first and second ends for the location of at least part of a user's hand digit in use, and wherein the first digit location means is provided along the length of the body portion between the first and second ends, and the at least second and third digit location means are provided either side of the first digit location means.

The arrangement of the digit location means on the gripping device according to the present invention provides an improved ergonomic design that allows a user to hold the gripping device without the risk of a user's hand slipping thereon, to allow a user to grip the device for extended periods of time without causing, or at least reducing, the likelihood of RSI type injuries, and to allow the user to have greater control and manoeuvrability of the device in use.

Preferably the first digit location means is provided along substantially the entire length of the body portion between the first and second ends thereof.

Preferably the first digit location means is designed and shaped to allow at least part of a user's index finger to be located therein in use, and further preferably the palm or rear side of the user's index finger. Since the length of the first digit location means preferably runs substantially the entire length of the gripping device, this allows the index finger to be substantially fully supported along its length in use if required. This provides a greater level of support for the finger and improved control of the gripping device caused at least partially by the index finger. This is in contrast to the prior art wherein only the finger tip of the palm side of the index finger is supportable in a recess of the gripping portion in use.

The digit location means are preferably shaped and/or dimensioned to fit an average sized user's hand, fingers and/or digits.

In one embodiment the central axis or longitudinal axis of the first digit location means, which can for example be in the form of a recess, groove or channel, is non-linear along substantially its entire length. Thus, in this embodiment the first digit location means may have a central axis that differs in angle along the length of the location means. For example, the first location means can have a degree of curvature along its length.

Preferably the first digit location means has one or more side walls. Further preferably said one or more side walls are not of the same or equal height along the length or longitudinal axis of said first digit location means.

Preferably a central plane, axis or longitudinal axis of the first digit location means, or at least a major portion of the first digit location means, is substantially parallel with or located at an angle less than 45 degrees to the longitudinal or central axis of the body portion. Further preferably the angle is between 20-30 degrees. Yet further preferably the angle is 25 degrees +/- 5.0 degrees, and further preferably still the angle is 25 degrees +/-2.5 degrees.

When a user's index finger is positioned in the first digit location means in use, if an approximate central plane or axis is taken through the user's finger from the finger tip to the first knuckle/joint below the finger tip, the angle of this central plane or axis with respect to the longitudinal axis of the body portion is substantially parallel with or located at an angle less than 45 degrees to the longitudinal axis of the body portion. Further preferably the angle is between 20-30 degrees. Yet further preferably the angle is 25 degrees +/- 5.0 degrees and further preferably still the angle is 25 degrees +/- 2.5 degrees.

The longitudinal axis of the body portion typically runs between the first and second ends and is preferably substantially central of the body portion and/or central or co-axial to a longitudinal axis of a tool located or locatable through the body portion or attached or attachable to the gripping portion. Preferably the first and second ends of the body portion are substantially opposite to each other.

Preferably the second digit location means is for the location of at least part of a user's thumb in use and further preferably for the location of a palm or rear side of the user's thumb.

In one embodiment the central axis or longitudinal axis of the second digit location means, which can for example be in the form of a recess, groove or channel, is non-linear along substantially its entire length. Thus, in this embodiment the second digit location means may have a central axis that differs in angle along the length of the location means. For example, the second location means can have a degree of curvature along its length.

Preferably the second digit location means has one or more side walls. Further preferably said one or more side walls are not of the same or equal height along the length or longitudinal axis of said second digit location means.

Preferably a central plane or longitudinal axis of the second digit location means, or at least a major portion of the second digit location means, is at an acute angle or at an angle of less than 90 degrees with respect to the longitudinal or central axis of the body portion. Further preferably the central plane or longitudinal axis of a major portion of the second digit location means is at an angle of between 30-80 degrees and yet further preferably is an angle of approximately 60 degrees +/- 6.0 degrees or less with respect to the longitudinal axis of the body portion. Further preferably still the angle is approximately 60 degrees +/- 3.0 degrees.

When a user's thumb is positioned in the second digit location means in use, if an approximate central plane or axis is taken through the user's thumb between the first and second thumb knuckles/joints below the nail, the angle of this central plane or axis with respect to the longitudinal or central axis of the body portion is at an acute angle or at an angle of less than 90 degrees with respect to the longitudinal or central axis of the body portion. Further preferably the central plane or longitudinal axis of a major portion of the second digit location means is at an angle of between 30-80 degrees and yet further preferably is an angle of approximately 60 degrees +/- 6.0 degrees or less with respect to the longitudinal axis of the body portion. Further preferably still the angle is approximately 60 degrees +/- 3.0 degrees.

Preferably with the user's fingers in position on the gripping device in use, the location means are arranged such that the tip of the user's thumb in the second digit location means is behind or set back from the tip of the user's index finger in the first digit location means.

Preferably the body portion has a front edge or surface and a rear edge or surface and the second digit location means is provided along substantially the entire side wall between the front and rear edges or surfaces.

Preferably the third digit location means is for the location of at least part of a user's middle finger in use. Further preferably the third digit location means is arranged for the location of a side and/or front of the user's middle finger.

In one embodiment the central axis or longitudinal axis of the third digit location means, which can for example be in the form of a recess, groove or channel, is non-linear along substantially its entire length. Thus, in this embodiment the third digit location means may have a central axis that differs in angle along the length of the location means. For example, the third location means can have a degree of curvature along its length.

Preferably the third digit location means has one or more side walls. Further preferably said one or more side walls are not of the same or equal height along the length or longitudinal axis of said third digit location means.

Preferably a central plane or longitudinal axis of the third digit location means, or at least a major portion of the third digit location means, is provided substantially transverse to the longitudinal axis of the body portion. Further preferably a central plane or longitudinal axis of a major portion of the third digit location means is provided between 65 -95 degrees. Yet further preferably the angle is 85 degrees +/- 8.5 degrees. Further preferably still the angle is 85 degrees +/- 5.0 degrees.

When a user's middle finger is positioned in the third digit location means in use, if an approximate central plane or axis is taken through the user's middle finger between the first and second finger knuckles/joints below the nail, the angle of this central plane or axis with respect to the longitudinal or central axis of the body portion is provided between 65-95 degrees. Yet further preferably the angle is 85 degrees +/- 8.5 degrees. Further preferably still the angle is 85 degrees +/- 5.0 degrees.

Preferably the body portion has a front edge or surface and a rear edge or surface and the third digit location means is provided along substantially the entire side wall between the front and rear edges or surfaces.

Preferably the side wall of the second digit location means is a first side wall and the side wall of the third digit location means is a second side wall. Further preferably the first side wall is substantially opposite to the second side wall.

Preferably the first digit location means is defined in a front surface of the body portion. Further preferably a front end or front open end of the second digit location means is adjacent to or abuts against the wall defining at least part of the first digit location means. Preferably a front end or front open end of the third digit location means is adjacent to or abuts against the wall defining at least part of the first digit location means.

Preferably the front end or front open end of the third digit location means that meets the first digit location means is substantially opposite to the front end or front open end of the second digit location means that meets the first digit location means.

With the user's digits in place on the body portion, if a central plane or axis is taken through the user's index and middle fingers between the hand knuckle/joint and the middle or next knuckle/joint of said fingers, the angle of said plane or axis for both fingers is substantially parallel or approximately 30 degrees +/- 5 degrees with respect to the longitudinal axis of the body portion. Further preferably the angle is approximately 30 degrees +/- 3 degrees.

Preferably the at least first, second and/or third digit location means are in the form of one or more recesses, grooves, channels and/or the like.

Preferably an interior or base surface of the recess, groove channel or digit location means is substantially curved or has a degree or curvature to allow the digit to more comfortably sit therein in use.

Preferably each of the digit location means is a contoured channel or recess. The contoured surfaces are preferably irregular in shape.

In one embodiment the side or second and third digit location means are preferably concave in shape. The top or first digit location means preferably has at least a convexed shaped section. In one embodiment the top or first digit location means also has a concave shaped section.

Preferably distinct edges and corners exist between the first, second and/or third digit location means.

Preferably eight or more discrete surfaces are defined on the device between the edges and corners provided thereon. The first, second and third digit location means typically comprise three of the eight discrete surfaces. Preferably the two ends of the device comprise a further two of the eight discrete surfaces. The remaining three of the eight discrete surfaces are typically defined between the first, second and/or third digit location means.

Preferably one end of the gripping device is substantially triangular in shape. This end is typically a second end or an end opposite a tattoo tube tip or first end in use.

Preferably one end of the gripping device is substantially nebulous in shape or most closely defined as a diamond shape. This end is typically a first end or an end in which a tattoo tube top is provided in use.

The gripping device can be integral with, detachably attached or attached to at least part of a tool or object to which the gripping device is to be used with in use. The tool or object is typically a hand held tool or object and the gripping device allows the tool or object to be held or gripped in a user's hand in use.

The gripping device can be attached or detachably attached to the tool or object via any suitable attachment means, such as for example via any or any combination of adhesive, welding, friction fit, one or more screws, nuts and bolts, clips, ties, interengaging members and/or the like.

Preferably one or more apertures, channels and/or slots are defined in the body portion for the location of one or more parts of a tool or object in use.

Further preferably the one or more apertures, channels and/or slots are defined in the first and/or second ends of the body portion.

In one embodiment a single aperture, channel or slot is defined between the first and second ends of the body portion (i.e. it extends throughout the body portion). A part of a tool or object is located through the aperture, channel or slot in use.

In a preferred embodiment the tool or object is in the form of a tattooing tube. The tattooing tube typically has a tip at a first or front end of the tube. An ink reservoir is typically located adjacent the tip and is preferably an open ink reservoir. The tattooing tube typically has a stem portion at a second or rear end of the tube. Different sized tips and/or first end portions of the tube can be used with the gripping device as required.

Preferably the gripping device is located between the first and second ends of the tube and further preferably between the ink reservoir and the rear stem portion of the tube.

Preferably a portion of the tube adjacent the first end thereof is arranged for location in the aperture, channel or slot defined in the first end of the gripping device. Preferably a portion of the tube adjacent the second end of the thereof is arranged for location in the aperture, channel or slot defined in the second end of the gripping device.

Preferably each of the first, second and third digit location means has a front end and said front end is located substantially adjacent to the end of the body portion integral with, attached or detachably to the part of the tattooing tube adjacent the front end thereof.

The gripping device can be integrally formed with the tattooing tube to provide a single piece tattooing tube. The gripping device can be integrally formed with a rear stem of a tattooing tube and a front end of the tube can be attached or detachably attached to the gripping device. The gripping device can be integrally formed with a front end portion of a tattooing tube and a rear stem of a tattooing tube can be attached or detachably attached to the gripping device. Alternatively, both the front end portion and the rear stem portion of the tattooing tube can be detachably attached or attached to the gripping device. It will be appreciated that the gripping device can therefore be provided as a single piece tattooing tube, a two-piece or at least a three-piece tattooing tube.

The gripping device can be retrofitted to an existing gripping device, tool, object and/or the like or provided in place thereof.

In one embodiment the gripping device is formed from any or any combination of materials capable of being autoclaved, sterilised, heated and/or the like to allow cleaning of the same. For example, the gripping device can be formed from any or any combination of metal, rubber, plastic, glass and/or the like.

The gripping device can be moulded, cast, machined worked and/or the like.

The gripping device can be disposable or non-disposable (re-useable) as required.

In one embodiment the gripping device is specifically designed for a right handed person or a left handed person. It is envisaged that two separate devices will need to be provided in this embodiment, one for a right handed person and one for a left handed person. This ensures the device is ergonomically matched to the handedness of the user.

Preferably the gripping device is asymmetric along both the longitudinal axis and/or the transverse axis.

In one embodiment a second or rear end of the gripping device, when gripped by a user in use, protrudes outwardly of the user's palm between the space defined between the user's thumb and index finger.

According to a second aspect of the present invention there is provided a method of using a gripping device, said gripping device including a body portion having a first end and a second end and at least first, second and third digit location means defined in said body portion between said first and second ends, said method including the step of a user locating a hand digit in each of the first, second and third digit location means to allow a user to grip the device in their hand, and wherein the first digit location means is provided along the length of the body portion between the first and second ends, and the at least second and third digit location means are provided either side of the first digit location means.

According to further independent aspects of the present invention there is provided a tattoo tube gripping device; a tattoo tube with gripping device; and a hand held gripping device.

The gripping device of the present invention is ergonomically designed to alleviate the stresses placed on one or more digits, the hand, the wrist and/or the forearm of a user gripping the device in use.

An embodiment of the present invention will now be described with reference to the accompanying figures, wherein:
Figure 1 is a front view of the gripping device in one embodiment of the present invention;
Figure 2 is a first side view of the gripping device in figure 1;
Figure 3 is a second side view of the gripping device in figure 1;

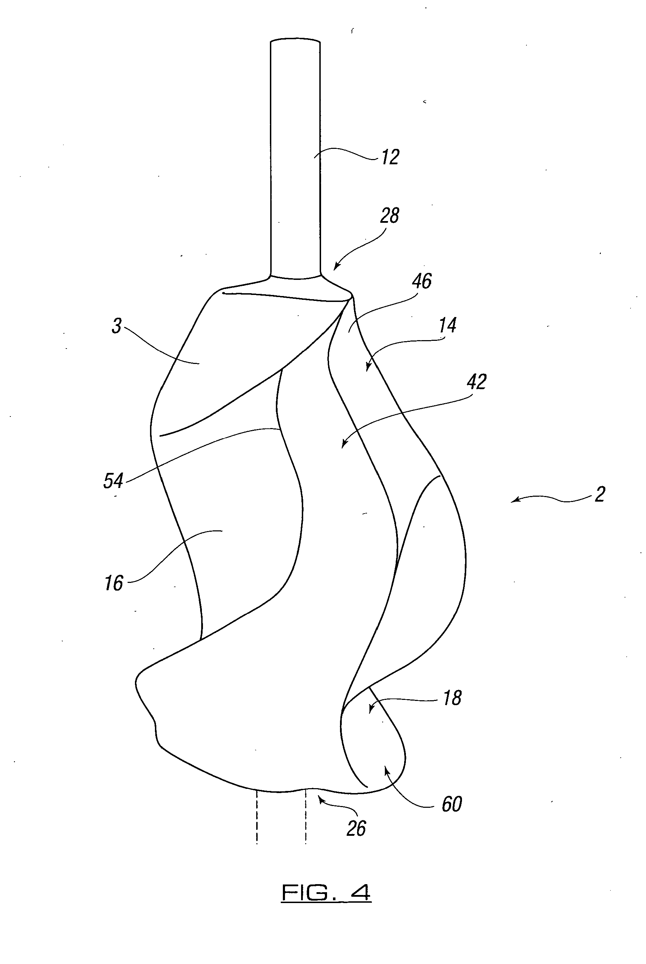

Figure 4 is a rear view of the gripping device in figure 1;

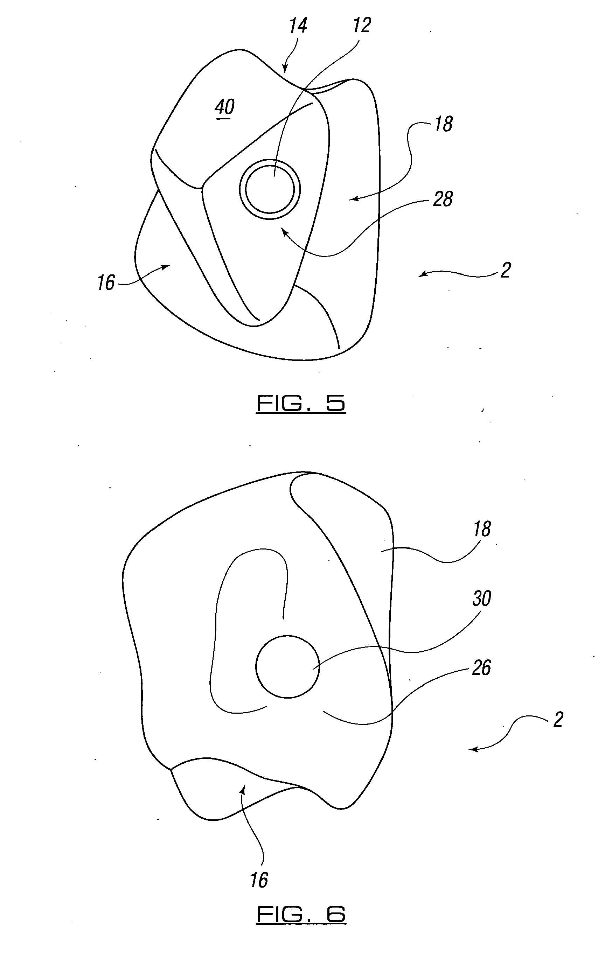

Figure 5 is a first end view of the gripping device in figure 1;
Figure 6 is a second end view of the gripping device in figure 1;

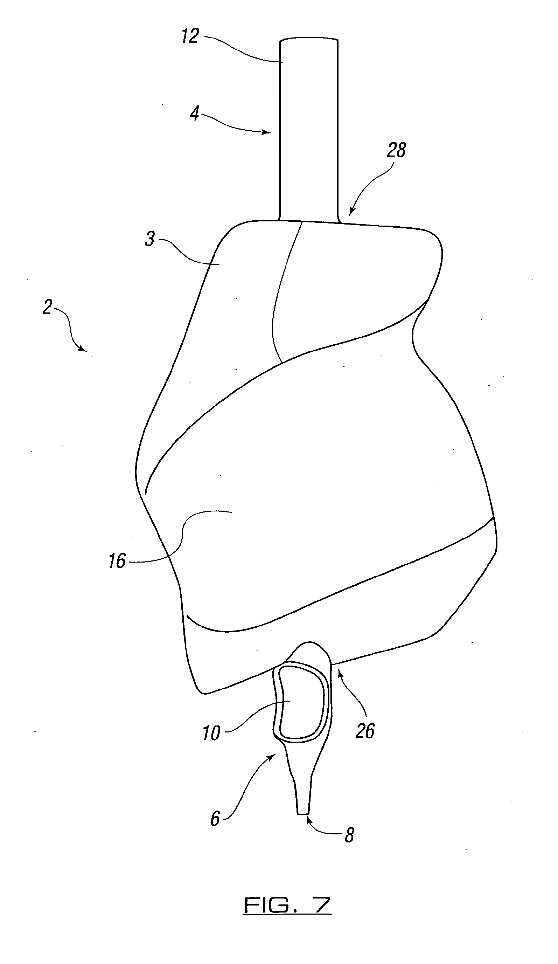

Figure 7 is a side view of the gripping device in figure 2 with a tattoo tube fitted through the same;

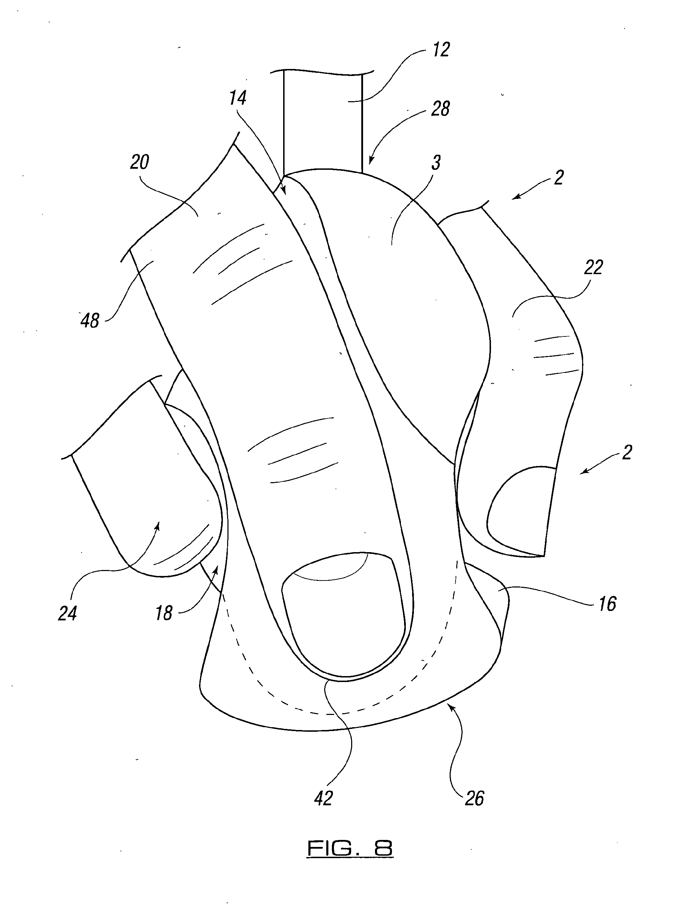

Figure 8 illustrates a user's digit position when gripping the gripping device shown in figure 1;
Figures 9a and 9b illustrate a side view and a front view of the gripping device in use showing the angles the user's fingers make with a central axis of the gripping device;
Figures 10a and 10b illustrate a side view and a front view of the gripping device in use showing the angle of the device to a worksurface when gripped by a user;
Figures 11a and 11b illustrate an opposite side view and rear view of the gripping device shown in figures 9a and 9b in use;

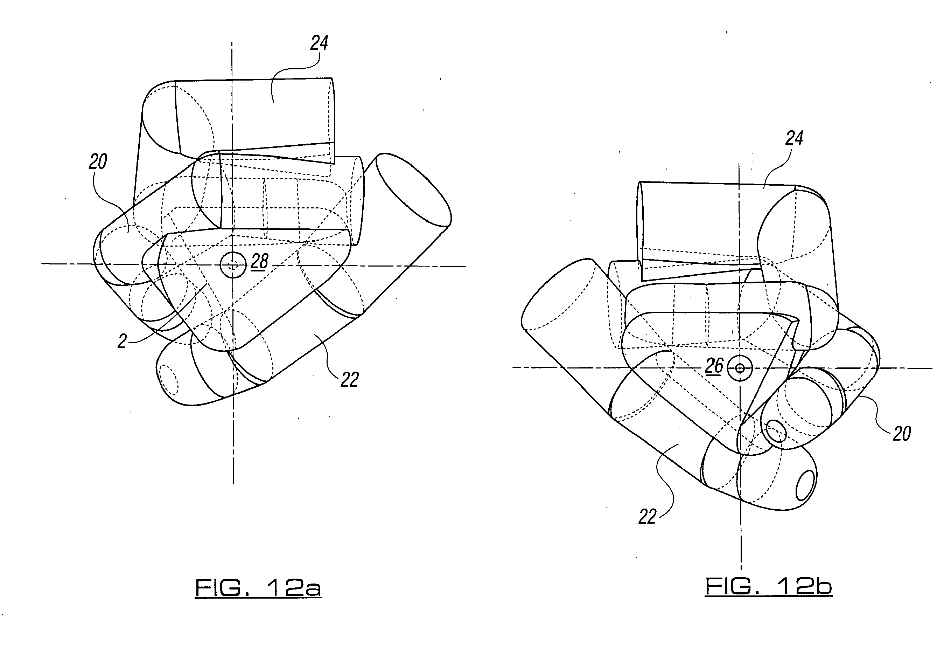

Figures 12a and 12b illustrate a top plan view and a base plan view of the gripping device in use showing the user's finger positions;
Figures 13a and 13b show further equivalent illustrations of the front view of the gripping device in figure 1 and first side view of the gripping device in figure 2 respectively with the position of the curves and recesses shown by solid lines;

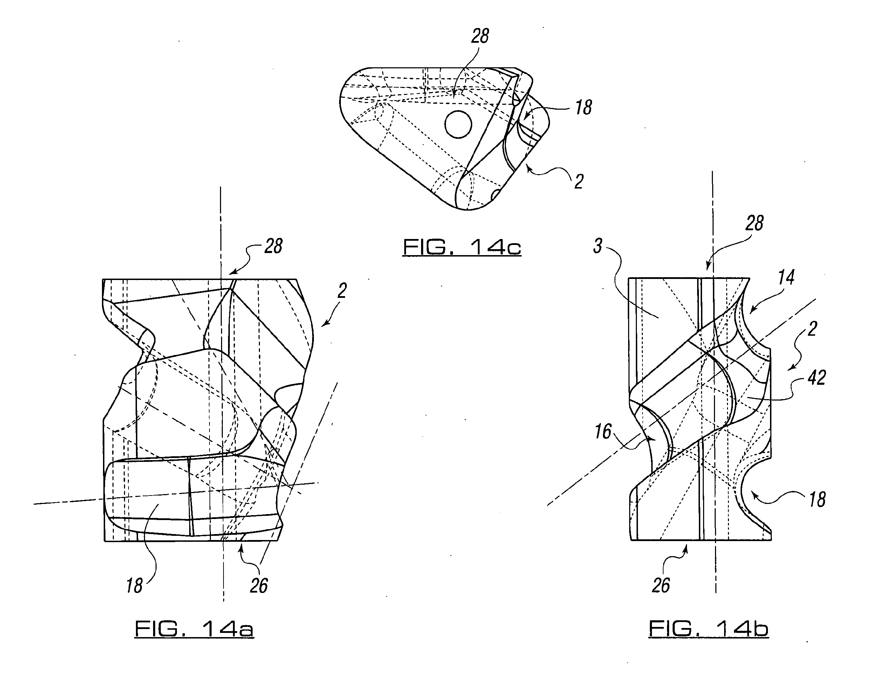

Figures 14a-14c show further equivalent illustrations of the second side view of the device in figure 3, rear view of the gripping device in figure 4 and first end view of the gripping device in figure 5 respectively with the position of the curves and recesses shown by solid lines;

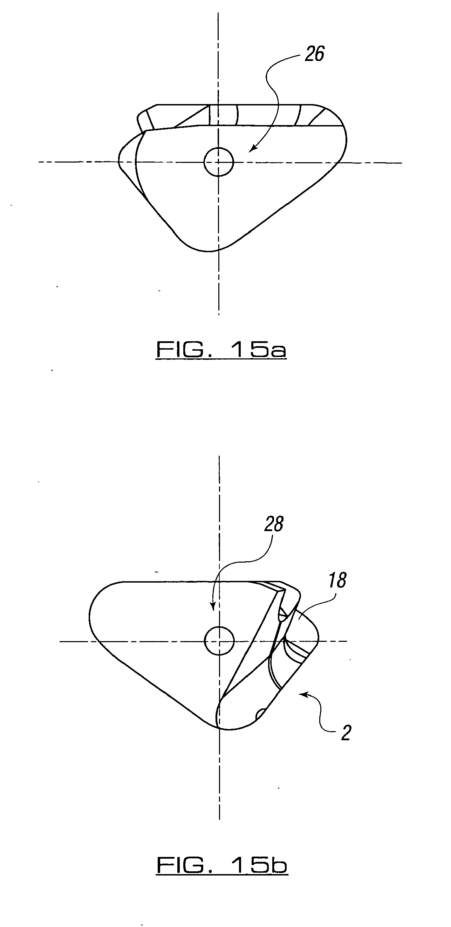

Figures 15a and 15b show a line drawing of the second end view and a first end view of the gripping devices in figures 6 and 5 respectively

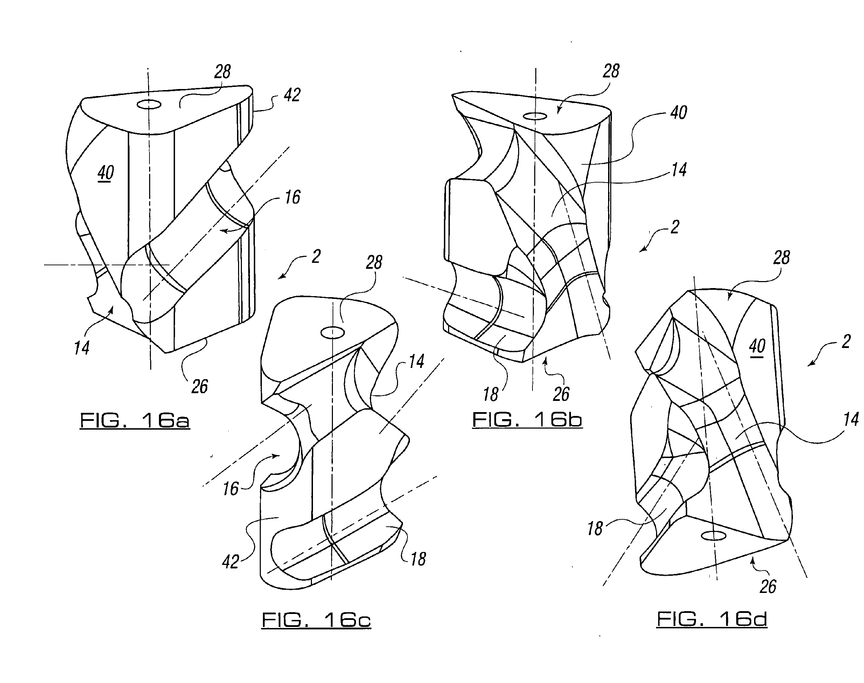

Figures 16a-16d are line drawings show a perspective front view of the gripping device from a first side; a perspective front view of the gripping device from a second side; a perspective rear view of the gripping device from the second side; and a front perspective view of the gripping device in figure 16a with the second end view of the device in view respectively.

Referring to the figures, there is illustrated an ergonomically designed gripping device 2 for use with a hand held tattooing tube 4. The tattooing tube 4 includes a first end portion 6 having a tip 8 at a free end thereof and an open ink reservoir 10 located adjacent tip 8 between the tip and the gripping device 2. Tattooing tube 4 also includes a second end portion in the form of a rear stem 12. The rear stem is typically attached to an electric tattooing device in use.

The gripping device 2 has a body portion 3 with three digit location means in the form of first, second and third elongate channels or recesses 14, 16, 18 defined therein respectively. The first channel 14 is for the location of a palm side of a user's index finger 20 in use. The second channel 16 is for the location of a palm side of a user's thumb 22 in use. The third channel 18 is for the location of the front and/or side of a user's middle finger 24 in use.

Body portion 3 typically has a first or front end 26 and a second or rear end 28. A channel 30 is defined through body portion 3 between first and second ends 28. In one embodiment, tattooing tube 4 is located through channel 30 and the front or first end portion 6 of tattooing tube 4 protrudes from the first end or front end 26 of body portion 3, and the second or rear stem portion 12 of tube 4 protrudes from the second end or rear end 28 of body portion 3 in use in one embodiment. It will be appreciated that any part or parts of the tattooing tube can be integrally formed with body portion 3, can be detached and/or detachably attached thereto.

The channel 30 is typically substantially parallel or co-axial with a longitudinal axis 34 of the body portion 3 or gripping device 2.

The first, second and third channels 14, 16, 18 all have a degree of curvature along their longitudinal axis to allow the digits to fit ergonomically therein in use. However, a substantially straight longitudinal or central axis/plane can be approximated through each of the channels to allow the position of the channels with respect to each other and to the longitudinal axis of the body portion to be more clearly defined herein.

If a number of virtual points were plotted at spaced apart intervals along the longitudinal axis of each channel at an approximate central location between the side walls thereof and a straight line was drawn that passed through as many of these virtual points as possible, this is typically considered to represent a major portion of the approximate central line or longitudinal axis of a channel.

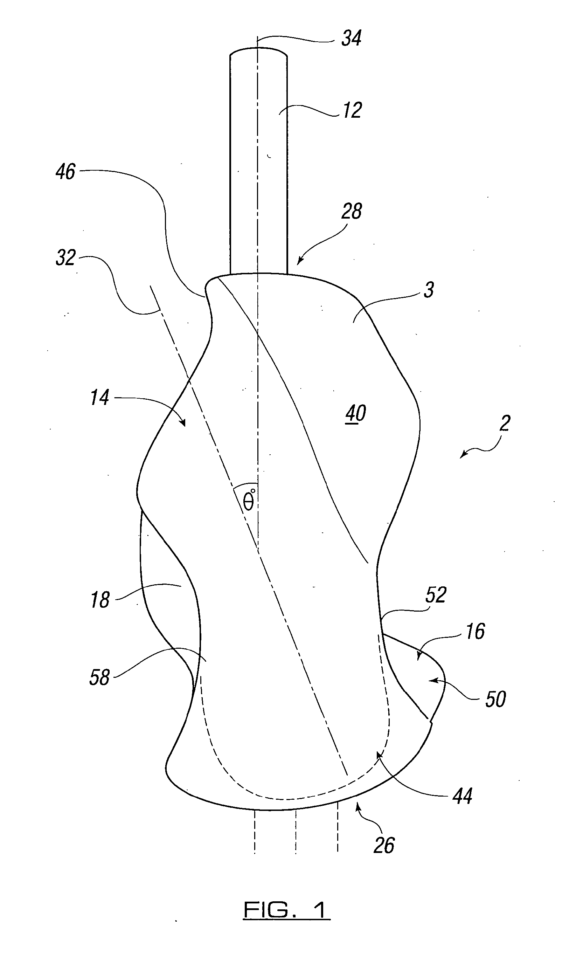

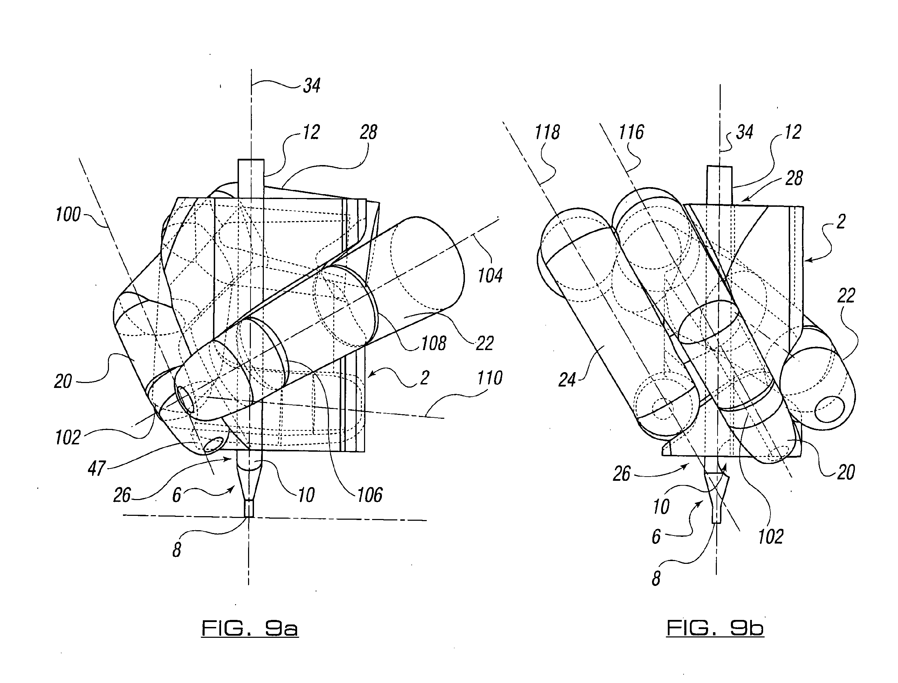

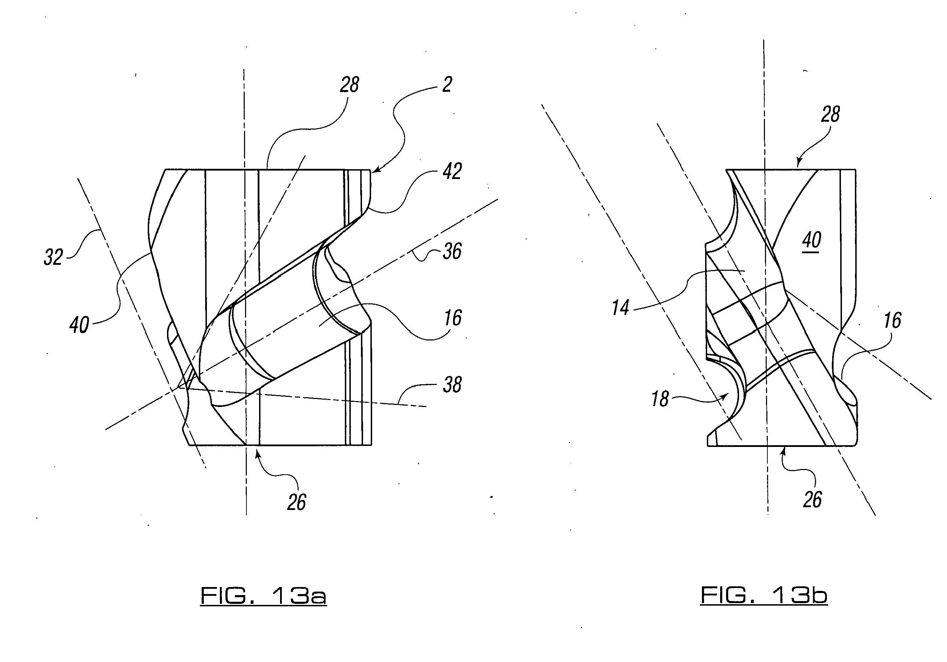

A major portion of the approximate central line or longitudinal axis 32 of first channel 14 is at an acute angle θ° to the longitudinal axis 34 of body portion 3, as shown in figure 1. This angle θ° is typically 25 degrees +/- 2.5 degrees, as shown in figure 13a. With a user's index finger 20 located in first channel 14 in use, if an approximate central line or longitudinal axis 100 is taken between the tip 47 of the index finger and the first knuckle/joint 102 below the tip, this axis 100 also typically forms an approximate angle of 25 degrees +/- 2.5 degrees with the longitudinal axis 34 of the body portion, as shown in figure 9a.

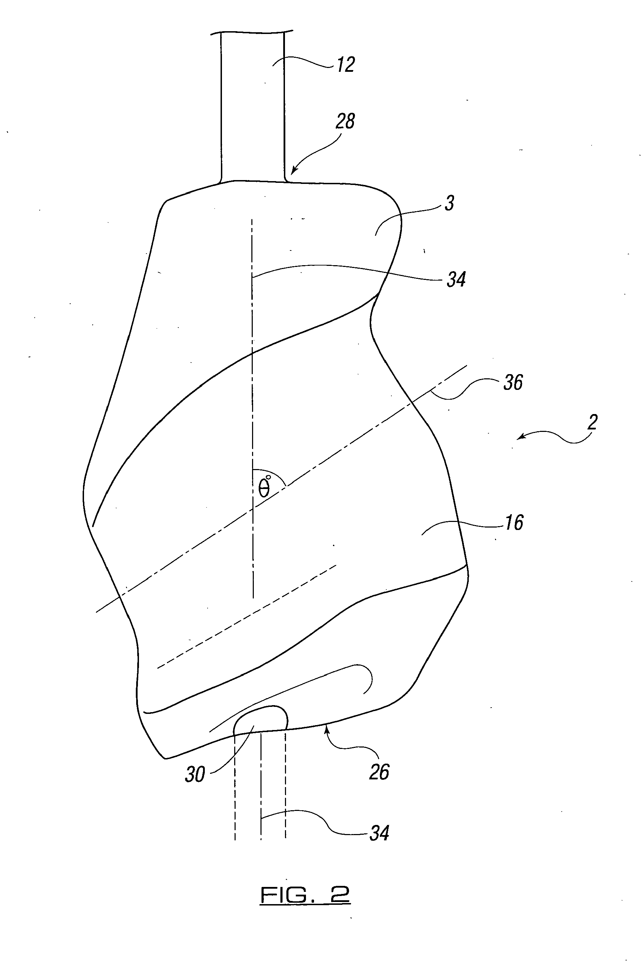

A major portion of the approximate central line or longitudinal axis 36 of second channel 16 is at an acute angle φ° to the longitudinal axis 34 of body portion 3, as shown in figure 2. This angle φ° is typically substantially equal to 60 degrees +/- 6 degrees, as shown in figure 13a. With a user's thumb 22 located in second channel 16 in use, if an approximate central line or longitudinal axis 104 is taken between the first knuckle 106 and second knuckle 108 of the thumb, this axis 104 typically forms an approximate angle of 60 degrees +/- 6 degrees to the longitudinal axis 34, as shown in figure 9a.

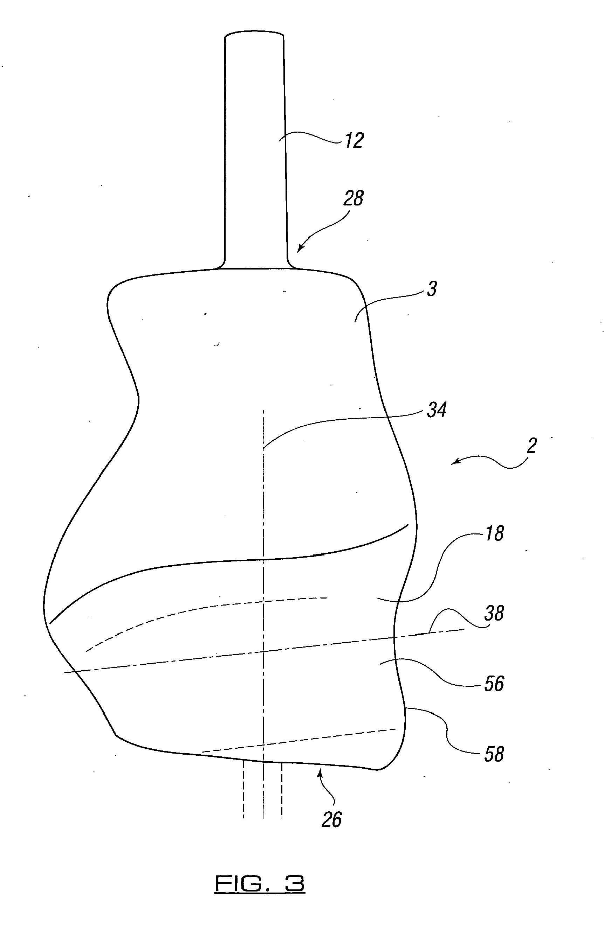

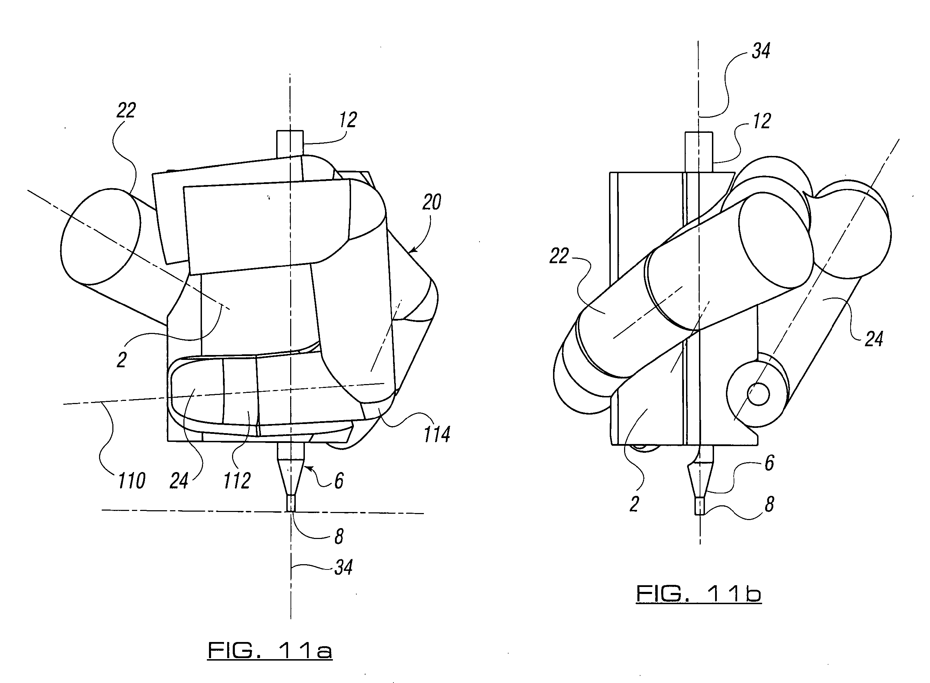

A major portion of the approximate central line or longitudinal axis 38 of third channel 18 is transverse and typically substantially at 85 degrees +/- 8.5 degrees to the longitudinal axis 34 of body portion 3, as shown in figures 3 and 13a. With a user's middle finger 24 located in third channel 18 in use, if an approximate central line or longitudinal axis 110 is taken between the first and second knuckles 112, 114 below the finger tip, this axis 110 typically forms an approximate angle of 85 degrees +/- 8.5 degrees to the longitudinal axis 34, as shown in figures 9a and 11a.

With the gripping device held in use by a user, the device typically has a front surface 40 and a rear surface 42.

The first channel 14, or at least a major part of the first channel 14, is defined on or across the front surface 40 of body portion 3. The first channel 14 is typically defined along substantially the entire length of body portion 3 from a first end 44 adjacent first end 26 of body portion 3 to a second end 46 adjacent second end 28 of body portion 3. This provides the user's index finger with almost continuous support within the channel 14, along the length thereof, and support along almost the entire length of the body portion, particularly between the tip 47 of the user's index finger to the user's knuckle 48. This provides the user with a greater level of support and control, thereby allowing the user to grip the device for longer periods of time compared to prior art devices.

The second channel, or at least a major portion of the second channel 16, is typically defined between the front and rear surfaces 40, 42 and typically across substantially an entire side wall of the gripping device 2. The length of the channel across the entire side provides elongated support for the user's thumb during gripping of the device. A first end 50 of second channel 16 is provided adjacent first end 26 of body portion 3. The first end 50 is typically a greater distance away from end 26 of body portion 3 compared to first end 44 of first channel 14. First end 50 is adjacent first end 44 and is separated by a ridge or edge 52. The second end 54 of second channel 16 is typically provided a spaced distance apart from second end 28.

The third channel, or at least a major portion of the third channel 18, is typically defined between the front and rear surfaces 40 42 and typically across substantially an entire side wall of the gripping device 2. The length of the channel across the entire side wall provides elongated and improved support for the user's middle finger during gripping of the device. A first end 56 of channel 18 is adjacent first end 44 of the first channel 14 and is separated by a ridge or edge 58. Both first end 56 and second end 60 of channel 18 are provided adjacent first end 26 of the body portion 3. The majority of the surface area of third channel 18 is provided nearer to first end 26 of body portion 3 compared to the majority of the surface area of second channel 16.

The width of the second channel (i.e. the distance transverse to the longitudinal axis of the channel) is approximately 2-3 times larger than the width between a lower edge of the second channel (i.e. the edge of the second channel closest to the first end of the body portion) and the first end of the body portion, or the width between a top edge of the second channel (i.e. the edge of the second channel closest to the second end of the body portion) and the second end of the body portion.

The asymmetrical location of the channels 14, 16, 18 provides an improved ergonomic design for a gripping device specific for either a right handed or a left handed person compared to prior art gripping devices. It has also been found by the applicant that when the gripping device is used with a tattoo tube, the user's fingers are positioned relative to the tattoo tube such that the weight of the electric tattooing device attached to the tattoo tube in use is more evenly positioned over the back of the user's hand, thereby allowing a user to have greater stability and control of the tattoo device in use. Thus, the second end of the gripping device protrudes outwardly of the user's palm in the gap between the user's thumb and index finger in use.

With reference to figure 9b, with the device in use, an approximate central or longitudinal axis or line 116, 118 taken between the hand knuckle/joint and the next knuckle/joint towards the finger tip of both the index finger 20 and the middle finger 24 is substantially parallel and is approximately 30 +/- 3 degrees with respect to the longitudinal axis 34 of the body portion.

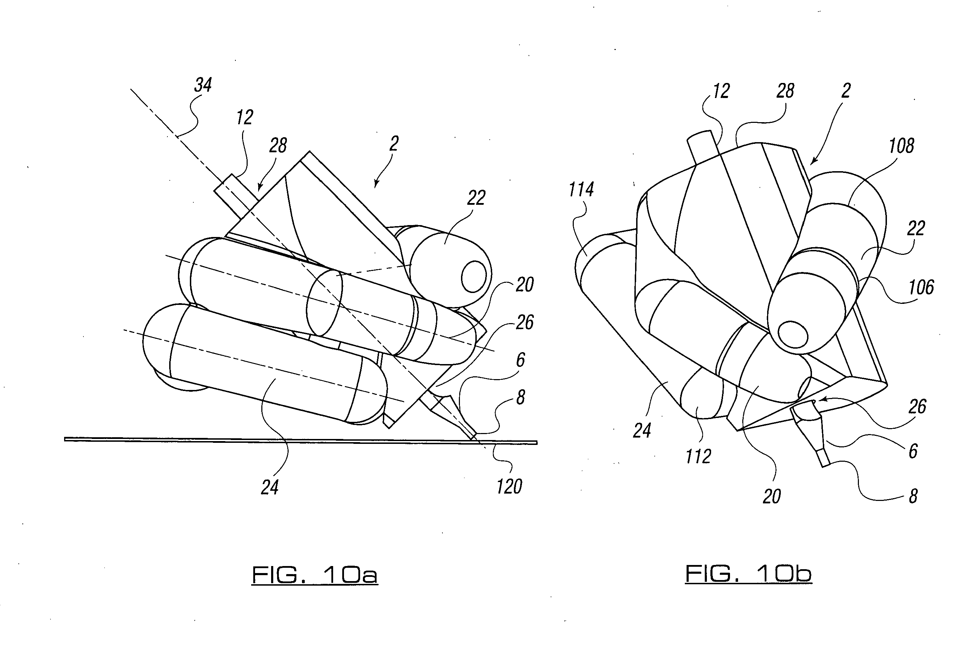

With reference to figures 10a and 10b, it can be seen that when the device is gripped in use, the preferred or average angle that the device 2 is held relative to a worksurface 120 is 45 degrees +/- 4.5 degrees but it will be appreciated that the user can select the required angle they wish to support the device at in use.

## Claims

1. A gripping device for gripping in a user's hand in use, said gripping device including a body portion having a first end and a second end and at least first, second and third digit location means defined in said body portion between said first and second ends for the location of at least part of a user's hand digit in use, and wherein the first digit location means is provided along the length of the body portion between the first and second ends, and the at least second and third digit location means are provided either side of the first digit location means.

2. The gripping device of claim 1 wherein the first digit location means is provided along substantially the entire length of the body portion between the first and second ends.

3. The gripping device of claim 1 wherein the first digit location means is of such dimensions and shape for location of at least part of a user's index finger therein in use, the second digit location means is of such dimensions and shape for location of at least part of a user's thumb therein in use, and the third digit location means is of such dimensions and shape for location of at least part of a user's middle finger therein in use.

4. The gripping device of claim 1 wherein a central plane, axis or longitudinal axis of the first, second and/or third digit location means is substantially non-linear.

5. The gripping device of claim 1 wherein the first, second and/or third digit location means has one or more side walls and the one or more side walls are not of equal height along the length thereof.

6. The gripping device of claim 1 wherein a central plane, axis or longitudinal axis of a major portion of the first digit location means is substantially parallel with or located at an angle less than 45 degrees to the longitudinal or central axis of the body portion, is between 20-30 degrees to the longitudinal axis or central axis of the body portion or is 25 degrees +/-5 degrees to the longitudinal axis or central axis of the body portion.

7. The gripping device of claim 1 wherein a central plane, axis or longitudinal axis of a major portion of the second digit location means is at an angle of less than 90 degrees with respect to the longitudinal or central axis of the body portion, is between 30-80 degrees to the longitudinal axis or central axis of the body portion or is 60 degrees +/-6 degrees or less to the longitudinal axis or central axis of the body portion.

8. The gripping device of claim 1 wherein the body portion has a front edge or surface and a rear edge or surface and the second digit location means is provided along substantially an entire first side wall between the front and rear edges or surfaces and/or the third digit location means is provided along substantially an entire second side wall between the front and rear edge or surfaces.

9. The gripping device of claim 1 wherein a central plane, axis or longitudinal axis of a major portion of the third digit location means is at an angle substantially transverse to the longitudinal or central axis of the body portion, is between 65-95 degrees to the longitudinal axis or central axis of the body portion or is 85 degrees +/-8.5 degrees to the longitudinal axis or central axis of the body portion.

10. The gripping device of claim 1 wherein the at least first, second and/or third digit location means are in the form of one or more recesses, grooves and/or channels.

11. The gripping device of claim 1 wherein an interior surface or base surface of the first, second and/or third digit location means is substantially curved or has a degree of curvature.

12. The gripping device of claim 1 wherein at least part of a tool or object to which the gripping device is to be used with in use is integral with, detachably attached or attached to the gripping device.

13. The gripping device of claim 12 wherein one or more apertures, channels and/or slots are defined in the body portion for the location of one or more parts of a tool or object in use.

14. The gripping device of claim 12 wherein the tool or object includes at least part of a tattooing tube.

15. A method of using a gripping device, said gripping device including a body portion having a first end and a second end and at least first, second and third digit location means defined in said body portion between said first and second ends, said method including the step of a user locating a hand digit in each of the first, second and third digit location means to allow a user to grip the device in their hand, and wherein the first digit location means is provided along the length of the body portion between the first and second ends, and the at least second and third digit location means are provided either side of the first digit location means.
